# EUROPEAN PATENT SPECIFICATION

(11) **EP 0 585 904 B1**
(45) Date of publication and mention of the grant of the patent: **26.11.1997**
(21) Application number: 93113985.1
(22) Date of filing: 01.09.1993
(51) Int. Cl.: A61F 13/15

(54) **Disposable diaper**
Wegwerfwindel
Couche jetable

(30) Priority: 02.09.1992 BR 9203486
(43) Date of publication of application: 09.03.1994
(73) Proprietor: McNEIL-PPC, INC., Milltown New Jersey 08850 (US)
(72) Inventor: Aledo, Eduardo Cesar Andreo, Sao Jose dos Campos (BR); Barrochelo, Antonio Carlos, Sao Jose dos Campos (BR)
(74) Representative: Strehl Schübel-Hopf Groening & Partner

(56) References cited:
- FR-A- 2 495 899
- FR-A- 2 573 629
- US-A- 4 892 536
- US-A- 5 037 413
- US-A- 5 062 840

## Description

The present invention is for disposable diapers aimed at avoiding or reducing leakage.

More specifically, it refers to disposable diapers comprising a physical structure which tends to inhibit the contact of the user's feaces with urine, creating an impounding to the feaces in a depression, at the same time imposing a physical barrier to its displacement along the surface of the diaper by the presence of an elevation ahead of the depression (see FR-A-2 495 899).

Disposable diapers are articles of diffused use, both for young aged children during the period of learning to control the physiological discharges of faeces and urine, as well as for incontinent people, for which this control is not complete.

They are articles that aim at only one utilization after which they are thrown away, without any procedure of recuperation. This aspect is appreciated in function of the practicalness and cleanliness obtained.

The disposable diapers are typically composed of a nucleus or absorbent panel which has two opposite sides, inside of a sandwich type structure which comprises a cover layer permeable to liquids, destined to remain in contact with the user's skin, turned to the first side of the absorbent nucleus, and another layer of lining impermeable to liquids in contact with the second side contrary to the absorbent nucleus, whose aim is to prevent leakage of the faeces and urine present there.

Such materials are known and dominated in the state of the art, making it unnecessary for greater definitions.

Such diapers may be endowed with several accessories to optimize their performance such as adhesive tapes for closure and adjustment to the user's body, lateral border elastics to make the conformation to the user's legs easy and to inhibit leakage, vertical elastic barrieres against leakage, etc.

Such materials, their accessories and their disposition in disposable diapers are exceedingly known by the technician and dispense further descriptions.

It is also known by the technician how difficult it is to create a disposable diaper which is able to absorb and contain simultaneously and effectively both faeces and urine discharged on it. The ways of dealing with faeces and urine are necessarily distinct, to begin with, by their physical states.

The means to deal specifically with the absorption of urine in disposable diapers are well known and quite frequent. For example:
- layers of absorbent material of different densities overlapping each other,
- absorbent structures containing hydrogels, which are materials capable of absorbing liquids in quantities many times superior to their own weight,
- absorbent structures with intermediary layers which increase the velocity of absorption of liquid in the horizontal plane, for the case of large volume discharges of urine,
- covering films with funneled orifices, permitting quick transference of the liquid from the external surface to the contiguous absorbent material, also inhibiting the return of the liquid to this surface.

Nevertheless, the functioning of such means are partially or totally jeopardized when the diaper suffers a discharge of faeces. The faecal material spreads itself in an aleatory fashion over the surface of the permeable material which recovers the absorbent nucleus of the diaper, obstructing it or making it impermeable so that a posterior discharge of urine or even more faeces has little possibility of penetrating and to have contact with absorbent material. In short, the desired absorption does not occur and favorable conditions for leakage are created.

This invention aims at eliminating or reducing to a great extent the problem of leakage in disposable diapers caused by the impermeability of the surface of the same by the discharge of faeces, a fact which permits that posterior discharges both of faeces and urine have great possibilities of leaking.

This is accomplished providing a disposable diaper with specific topography which provokes a physical barrier to the displacement of the faeces over the surface towards the front region. Such a barrier is possible by the existence of two regions in the surface or structure of the diaper, with opposite concavities, so that when put to use, the user's perineum rests between the two regions.

The first region (hereunto denominated as elevation) is located at the front of the user's perineum and is turned inward, i. e., the diaper is constrained in the direction of his body.

The second region (hereupon denominated as depression) is located at the rear part of the diaper and is turned in the direction contrary to the user's body, i. e., there is a pocket or sack imposed to the diaper to move itself away from the body.

Preferably, the user's perineum (region between the sexual organ and the anus) remains accommodated near the middle point between the elevation and depression comprised in the structure of the diaper, when the same is in use.

The depression in the rear region of the diaper is located near the point of the discharge of the user's faeces and aims at repressing the same by its conformation. Such repression implies a confinement of the faeces in this place, and such a confinement is even more effective by the close presence of the elevation, ahead of the perineum, which creates an additional physical barrier preventing the faeces from having its course unobstructed towards the front region, causing impermeability of the surface and jeopardizing the absorption of later discharges.

There are some attempts in the state-of-the-art technology which aim at restricting leakage in disposable diapers mentioned as follows.

The patent US 3,860,000 from Buell foresees disposable diapers with elastic brims to guard the user∩s legs, functioning as a physical barrier against overflow of liquid out of the absorbent panel. There is no prevision as to how to avoid the free course of the faeces, which causes for the surface to be impermeable, favoring leakage.

The patent 082,182,814 from Toussant and Blaney referring to a disposable diaper with flexible brims which, with their fold configuration, create lateral reservoirs for faecal material transported to them. There is no prevision as to how to avoid the free course of the faeces discharged over the surface.

The patents US 1,900,117, US 4,802,500 and the Patent Application EP 386 816 from Procter & Gamble divulge diapers that comprise a layer of covering not adherent to the absorbent nucleus, said layer provided with a passage for the faeces in the central region. When in use, the diaper configures a type of hold between the covering and the absorbent nucleus turned to the reception of the faeces. Such an arrangement aims at preventing the contact of the discharged faeces with the user's body, but it does not restrict the free course of the faeces over the surface of the absorbent material towards the front region.

One of the objectives of this invention is to eliminate or remedy substantially the leakage in disposable diapers.

Another objective of the present invention is a disposable diaper which limits the mixture between faeces and urine discharged over the surface, so that it permits the exemption of faeces in certain areas of the surface of the diaper.

Still another objective of this invention is a disposable diaper which limits the contact of faeces with the userÿs genital organ, reducing the damaging potential of this contact, through the confinement of the faeces.

Still another objective of this invention is a disposable diaper which, by its effectiveness in the separation between faeces and urine, offers greater comfort to the user.

One more objective of the present invention is a disposable diaper of high performance against leakage and low manufacturing cost.

These and other objectives from the present invention are better understood along the description which follows, with the help of the attached drawings.

A disposable diaper which corresponds to the teachings of this invention is typically an article for children's use, but it may also be used for incontinent people of any age, varying the dimensions of the article.

The present invention of a disposable diaper characterized by comprising regions of elevation and depression, of opposite concavities, so that when the said diaper is put to use, the user∩s perineum is located between the two such regions, being the elevation region anterior and constrained at the encounter of the user's body, and the depression region posterior and constrained in contrary direction to the user's body.

The anterior and posterior regions are mentioned in order to make reference, respectively, to the front and rear parts of the user's body.

As employed here, the terms "elevation" and "depression" refer to the diaper of the invention when imagined open over a horizontal plane in such a way that one may define a quota difference between any two points of the surface of the diaper, relatively to an imaginary axis vertical to such a plane. The plane mentioned refers to the zero quota of the said vertical axis, above it, the quotas are positive, and below it, the quotas are negative. In this way, the reference to "elevation" refers to any geometric figure whose base is located substantially over the said plane of the diaper and its extremity, punctual or not, in some quota of positive value; similarly, the term "depression" refers to any other geometric figure whose base is located substantially over the said plane of the diaper and its extremity, punctual or not, in some negative quota, therefore configuring a concavity contrary to the "elevation".

Still as employed here, "to constrain" a material has the meaning of obtaining determined conformation - notedly in respect to depression and elevation mentioned - and a determined positioning in relation to the user's body, without necessarily denoting association with elastic forces or any other nature to obtain such an effect.

Also as employed here, "elevation" is a region more elevated than the plane of the surface of the diaper, having, therefore, a positive quota, and "depression" is a lower region in relation to the same plane, having, therefore, negative quota so that when the diaper is put to use, the elevation is located to the front of the user and turned in direction of his body, and the depression has a posterior location to the user's body and turned in the contrary direction of his body. When such a diaper is put to use, the user's perineum is located substantially between the said elevation and the said depression.

Preferentially, the diaper of the invention comprises at least one elevation and one depression along the surface and/or the structure of the diaper.

The location of the elevation (or the system elevation-depression) is not necessarily symmetrical in relation to the dimensions of width and length of the diaper.

Preferably, the localization is symmetrical in relation to the lateral borders of the said diaper. The technician of the matter knows where to best bring about such location, keeping in mind the ergonometrics of the use aimed at, to attain comfort and efficiency.

The elevation here mentioned may be obtained by any mean, for example, through a stuffed protuberance of filling material, absorbent or not, or still a similar protuberance to a hollow hub cap inside, preferably flexible, conformed as desired, forcing the contact of the diaper with the user's body.

Preferentially, elastic means are utilized associated to the structure of the diaper during use to obtain deformation of the same corresponding to the elevation described. One of the advantages of this preferential embodiment is the fact that it tends to reduce the distance between diaper and user's body by means of its elastic action regardless of the user's movement, disfavoring leakage. Such elastic means are any, including elements which are intrinsically elastic, for example, elastic filaments, or even those that become elastic by heating, for example, a fine film of polyvinylchloride PVC or ethylenevinylacetate EVA or still for example, foams or compound materials which possess, once stretched, the tendency to return to their original length.

Such elastic means may be associated to the diaper by any mean (adhesion, sewing, etc.) and they are located internally or externally to the structure of the diaper. Preferentially, they are located externally to the structure of the diaper, for example, fixed to the impermeable layer or lining.

Also preferably, the elevation in contact with the user's body is covered with absorbent material. The elevation may still be obtained, for example, by means of non-elastic pleats imposed to the lining of the diaper, consequently deforming the structure of this as desired.

The depression here described may be obtained by any means so as to permit that the same act as a represser of the faeces there deposited, or conducted to there. For example, such a repressing function is obtained by utilizing a receptacle in the form of a bulge. Preferably one or more elastic elements are utilized associated to the structure of the diaper during the use of the same, to obtain such a deformation that configures the depression described.

Preferentially, the depression is covered internally, on the side in contact with the user's body, with an absorbent material.

As already mentioned, the effectiveness of the diaper of the invention in minimizing leakage is due to the fact that it offers a repression of the faeces in the depression of the rear part of the said diaper, and additionally, limitations are created to the spreading of the faeces in direction of the anterior part by the physical barrier or elevation in front of the user's perineum.

With reduced chances of the faeces moving along the surface of the diaper, there will be in consequence, smaller chances of an obstruction or impermeability occurring on this surface, preserving the rest of the diaper, mainly the anterior part of the same for only urine absorption. Consequently, there will be fewer possibilities of leakage, greater comfort and assurance.

When put in use, the diaper of the invention involves a part of the user's lower abdomen with the front part going from the region of the waist near the navel to the region between the legs; the rear part begins there proceeding to the back in the region of the waist, over the rump.

The functioning of the diaper of the invention is especially advantageous when the diaper is worn on the user in a way that his or her perineum rests between the mentioned elevation and depression.

The existing elevation in the front part of the diaper of the invention may have any suitable format for the finality aimed at, which is to create a physical barrier against the advance of the faeces deposited at the posterior part of the diaper. For example, one may utilize a single protuberance, with an approximately conical shape located over an imaginary longitudinal central line of the diaper.

Also, the volume of the said elevation is any, turned to the function to which it is aimed.

In a similar fashion, format and volume of the depression are any as long as they serve the basic function of repressing the faeces.

As for the characteristics of the elevation and depression of the diaper of the invention, such as format, volume, flexibility, material, etc., it is left up to the technician to determine them, also keeping in mind the aesthetics and comfort desired for this type of article.

With relation to the distance between the elevation and depression of the diaper of the invention, it is advantageous that they be contiguous to attain the maximum cumulative effect of the difficulties imposed to the course of the faeces in direction of the anterior part of the diaper, i. e., the depression which represses and the elevation which creates a physical barrier to the displacement of the faeces.

Nevertheless, elevation and depression may be oposed without this limiting the scope of the invention. For example, one may differentiate a diaper for men from one for women, keeping in mind the perineum characteristics of each sex, mainly in adulta.

The disposable diaper of the invention may have any formats - rectangular, triangular, hourglass, etc. - and its structure may have any quantity and quality of layers, with the use of the most varied materials, for example, superabsorbents, zeolites, silica, etc., without this losing scope of the invention.

### SUMMERIZED DESCRIPTION OF THE DRAWINGS

Fig. 1 shows a schematic view in perspective of a particular accomplishment of the diaper of the invention, before being put to use.

Fig. 2 shows a longitudinal cut of the diaper of Fig. 1 made by an imaginary vertical plane along line XX, when put to use.

Fig. 3 shows a longitudinal cut of the diaper of Fig. 1 made by an imaginary vertical plane alonge line XX, before being put to use.

Fig. 4 shows a longitudinal cut similar to the one of Fig. 3 of an alternative accomplishment of the diaper of the invention.

Fig. 5 shows a transversal cut in perspective of the diaper of Fig. 1, made by an imaginary vertical B plane.

### DETAILED DESCRIPTION OF THE DRAWINGS

The drawings here attached are merely illustrative, with dimensions which may be distorted in relation to the real article, because the finality is only to make it easier to comprehend the invention. Such a fact does not impose any restriction to the scope of the invention, delimited only by the attached claims.

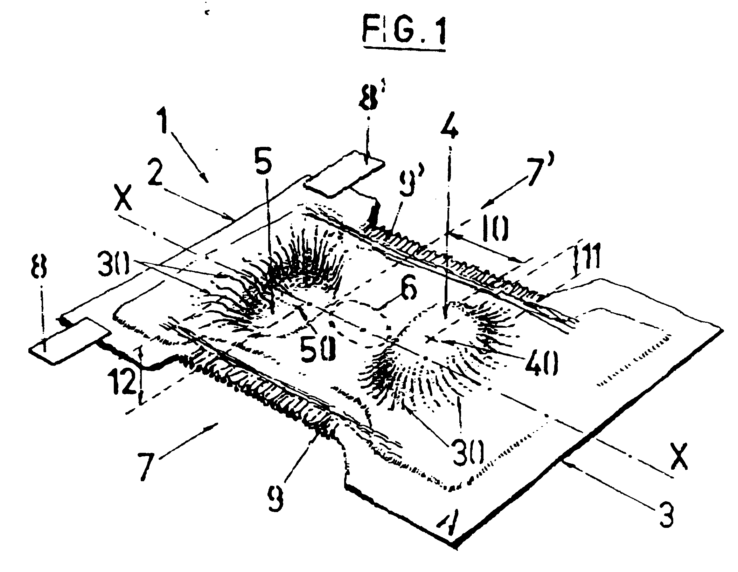

Fig. 1 shows in perspective, in a schematic way, a configuration of the disposable diaper according to the invention (within many variations which are possible).

Diaper 1, when not in use, is shown laid on a plane A and comprises a rear region 2, a front region 3, laterals 7 and 7', fixation tapes 8 and 8' and lateral elastic borders 9 and 9'. The contour of the diaper 1 gives it a form commonly called "hourglass", i.e. the region between the legs is narrower than the rest of the diaper, adapted to the user's anatomy.

Diaper 1 still comprises:
- an elevation 4, with an apex 40 turned inward, i.e., in direction to the user's body, away from plane A of the structure of the diaper at a distance 11. Its aim is to constrain the diaper against the user's body, creating a barrier to the displacement of the faeces in direction to the front part of the same;
- a depression 5 with an apex 50 away from plane A of the structure of the diaper by a distance 12 and turned in direction contrary to the apex 40, i.e., in direction contrary to the user's body. It is aimed at repressing the faeces there deposited or to there conducted.

Elevation 4 and depression 5 are away by a distance 10, and between them, a perineal region 6 is located aimed at receiving the user's perineum.

In the configuration presented, elevation 4 and depression 5 are conformed as such by the deformation of the structure of the diaper 1 in view of the contraction of elastic filaments 30 fixed radially to the periphery of 4 and 5.

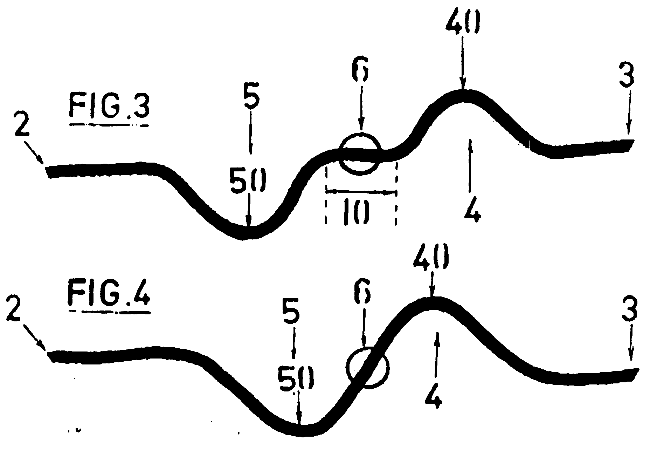

As may be seen in Fig. 3, when the diaper 1 is put to use wropping around the user's low abdomen 100, depression 5 remains near the user's anus and takes on the conformation of the bottom of a sack in a way to repress the faeces there deposited. The dotted line 5' shows how a diaper known in the state of the art without the possibility of repressing the faeces effectively.

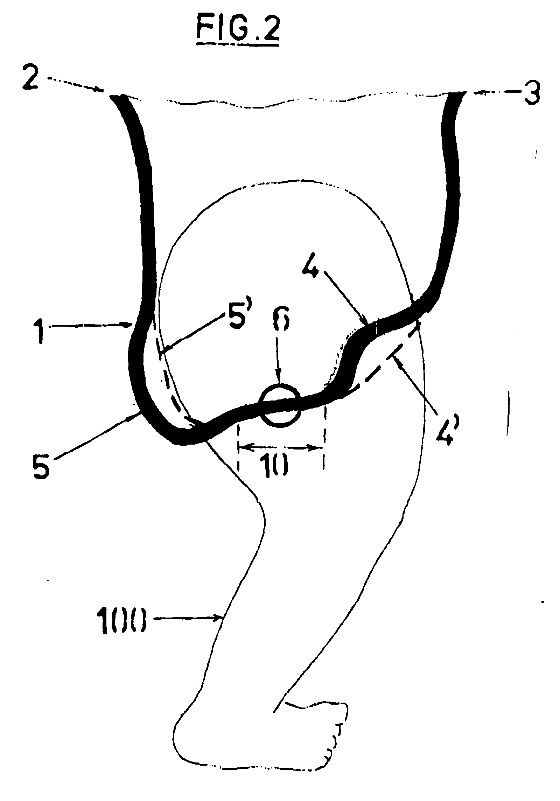

Still in Fig. 2, it is seen that the user's perineum rests naturally on the perineal region 6, and elevation 4 forces the diaper against the user's body. The dotted line 4' shows what the position would be like assumed by the front region of a diaper known the state of the art.

Elevation 4 is placed as a barrier between the posterior part of the diaper and the front part, preventing the free displacement of the faeces, even in situations of the user's movement.

It is easy to see in Fig. 2 that the course of the faeces between 5 and 4 is less probable than between 5' and 4', in function of the barriers imposed simultaneously by the depression 5 and elevation 4. Without the occurrence of such a course, the front part of the diaper is not blocked by the faeces, always being available for the absorption of urine, improving, in a general way, the performance and the comfort offered by the diaper.

Fig. 3 shows schematically a cut along a vertical plane (not represented) which cuts the diaper along axis XX shown in Fig. 1.

The perineal region 6 of Fig. 3 is located along the distance 10 between elevation 4 and depression 5. The distance 10 may not exist, according to the alternative configuration shown in Fig. 4, where the perineal region 6 is located between the apexes 40 and 50, preferably in the middle region between them.

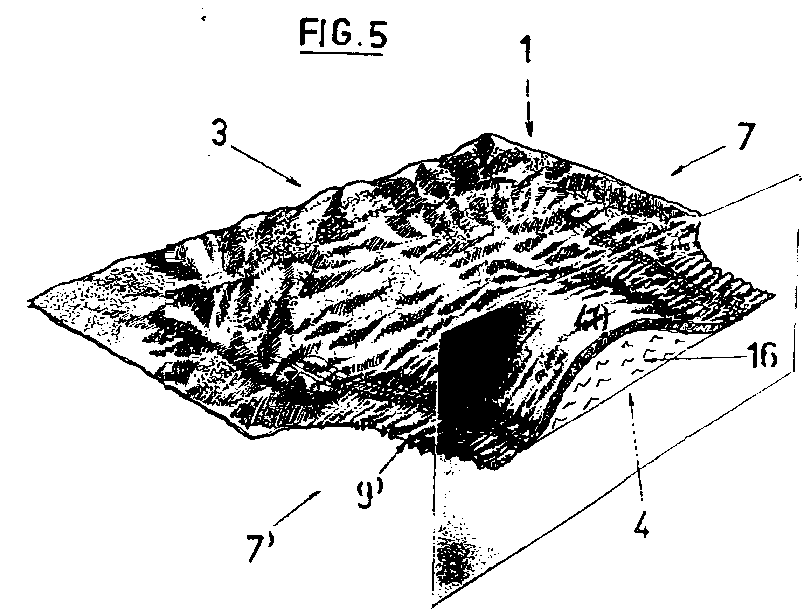

In another configuration of the invention, Fig. 5 shows, through a cut made by plane B transversal to the diaper 1 over elevation 4, the presence of a filling 16 which supports elevation 4, forcing it in the direction of and preferably to the encounter of the user's body. Filling 16 may be of the same quality as the absorbent material of the diaper 1, or it may be of any other material that behaves in a way that contrains the diaper against the user's body.

## Claims

1. Disposable diaper (1) having a rear region (2), a front region (3), laterals (7, 7') and a perineal region (6) comprising a physical structure that limits the contact of faeces and urine and that limits the contact of faeces with the user's genital organs
**characterized in that** said structure comprises regions of elevation (4) and depression (5), of opposite concavities whereby said perineal region (6) is located between said elevated (4) and depressed regions (5) and whereby said elevated region (4) being located anteriorly of said perineal region (6) towards the front region (3) and is constrained at the encounter of the user's body, and whereby said depressed region is located posteriorly of said perineal region (6) towards the rear region (2) and constrained in the direction contrary to the user's body.

2. Disposable diaper according to claim 1 characterized in that the said elevation and depression are arranged without space between them.

3. Disposable diaper according to claim 1 characterized in that the said depression is lined with absorbent material.

4. Disposable diaper according to claim 1 characterized in that the conformation of the said elevation and depression is obtained by elastic means associated to the structure of the diaper.

5. Disposable diaper according to claim 4 characterized in that the elastic means are fixed externally to the structure of the diaper.

## Patentansprüche

1. Wegwerfwindel (1) mit einem hinteren Bereich (2), einem vorderen Bereich (3), Seiten (7, 7') und einem perinealen Bereich (6), umfassend eine physische Struktur, die die Berührung mit Faeces und Urin begrenzt und die den Kontakt der Faeces mit den Geschlechtsorganen des Benutzers begrenzt,
dadurch gekennzeichnet, daß die Struktur erhöhte (4) und vertiefte (5) Bereiche sich gegenüberliegender Konkavitäten umfaßt, wodurch der perineale Bereich (6) zwischen den erhöhten (4) und vertieften (5) Bereichen angeordnet ist und wodurch der erhöhte Bereich (4) vor dem perinealen Bereich (6) in Richtung des vorderen Bereichs (3) angeordnet ist und beim Auftreffen auf den Körper des Benutzers eingezwängt wird, und wodurch der vertiefte Bereich hinter dem perinealen Bereich (6) in Richtung des hinteren Bereichs (2) angeordnet ist und in der zum Körper des Benutzers entgegengesetzten Richtung eingezwängt wird.

2. Wegwerfwindel nach Anspruch 1, dadurch gekennzeichnet, daß die Erhöhung und Vertiefung ohne Zwischenraum zwischen ihnen angeordnet sind.

3. Wegwerfwindel nach Anspruch 1, dadurch gekennzeichnet, daß die Vertiefung mit absorbierendem Material ausgekleidet ist.

4. Wegwerfwindel nach Anspruch 1, dadurch gekennzeichnet, daß die Anpassung der Erhöhung und Vertiefung durch elastische Mittel erreicht wird, die der Struktur der Windel zugeordnet sind.

5. Wegwerfwindel nach Anspruch 4, dadurch gekennzeichnet, daß die elastischen Mittel an der Struktur der Windel außen befestigt sind.

## Revendications

1. Couche jetable (1) présentant une région arrière (2), une région avant (3), des régions latérales (7, 7') et une région périphérique (6) comprenant une structure physique qui limite le contact des matières fécales et de l'urine et qui limite le contact des matières fécales avec les organes génitaux de l'utilisateur, caractérisée en ce que ladite structure comprend des réglons d'élévation (4) et de dépression (5), de concavités opposées, si bien que ladite région périnéale (6) est située entre lesdites régions d'élévation (4) et de dépression (5) et que ladite région d'élévation (4) située avant ladite région périnéale (6) vers la région avant (3) est comprimée contre le corps de l'utilisateur, et si bien que ladite region ce dépression située après ladite région périnéale (6) vers la région arrière (2) est comprimée dans la direction contraire au corps de l'utilisateur.

2. Couche jetable selon la revendication 1, caractérisée en ce que lesdites régions d'élévation et de dépression sont agencées sans espace entre elles.

3. Couche jetable selon la revendication 1, caractérisée en ce que ladite région de dépression est doublée d'un matériau absorbant.

4. Couche jetable selon la revendication 1, caractérisée en ce que la conformation desdites régions d'élévation et de dépression s'obtient par des moyens élastiques associée à la structure de la couche.

5. Couche jetable selon la revendication 4, caractérisée en ce que les moyens élastiques sont fixés à l'extérieur de la structure de la couche.
